# EUROPEAN PATENT APPLICATION

(11) **EP 4 717 758 A1**
(43) Date of publication of application: **01.04.2026**
(21) Application number: 24835781.6
(22) Date of filing: 08.05.2024
(51) Int. Cl.: C12M 1/00, C12Q 1/02, G01N 35/10

(54) **PROCESSING DEVICE AND PROCESSING METHOD**

(30) Priority: 05.07.2023 JP 2023111076
(71) Applicant: FUJIFILM Corporation, Tokyo 106-8620 (JP)
(72) Inventor: KASAI, Yusuke, Ashigarakami-gun, Kanagawa 258-8577 (JP); OBA, Takahiro, Ashigarakami-gun, Kanagawa 258-8577 (JP)
(74) Representative: Hoffmann Eitle
(86) International application number: PCT/JP2024/017174
(87) International publication number: WO 2025/009259

(57) **Abstract**

A processing device is a processing device for performing processing of handling a cell or a sample for cell culture by using a holding member including at least one holding section that holds the cell or the sample for cell culture. The processing device includes a protective surface that faces the holding section. The protective surface has an area larger than a planar area of a compartment in which the holding section is disposed. The processing device includes a first fixing structure for fixing an object that accesses the holding section for the processing.

## Description

### BACKGROUND OF THE INVENTION

### 1. Field of the Invention

The technology of the present disclosure relates to a processing device and a processing method.

### 2. Description of the Related Art

As technologies related to processing devices for handling cells or samples for cell culture, the following technologies are known. For example, JP2021-179433A discloses a sample preparation device including a base, an accommodating structure that is adapted to accommodate a plurality of sample receptacles, and a pipetting operation guide that is fixed so as to be movable relative to the accommodating structure. The pipetting operation guide includes a plurality of pipetting guide structures that can be positioned relative to the plurality of sample receptacles.

JP2008-134066A discloses a dispensing device that dispenses a sample into a sample container comprising a detachable lid, in a state in which the lid is removed. The dispensing device comprises a dispensing head that moves relative to the sample container to dispense the sample into the sample container, a lid capturing section that is provided on the dispensing head and that captures the lid, and a lid capturing section driving unit that drives the lid capturing section to move the lid captured by the lid capturing section to a side of the dispensing head.

### SUMMARY OF THE INVENTION

In the commercial production of cell-based preparations, it is common to employ closed culture bags or large-scale bioreactors. In these methods, because the cells are processed entirely within a closed space, a risk of cell contamination due to foreign matter, bacteria, or viruses is low.

However, for example, in a case in which special processing that is difficult to automate by a machine is required, the closed system equipment as described above may not be able to handle the processing. In this case, since the processing of handling the cell or the sample for cell culture is performed in an open environment, the risk of cell contamination increases. The cell contamination is caused by the foreign matter that falls from above, in addition to infection with bacteria or viruses.

In order to prevent the foreign matter that falls from above from being mixed into the cell or the sample for cell culture, it is conceivable to use a lid or a microfilter in the culture container. However, during processing steps such as culture medium exchange and pipetting, it is common to open the lid or the microfilter to perform the work. In this case, the movement of the operator's hand or a processing instrument above the cell or the sample for cell culture may cause the foreign matter to fall into the cell or the sample for cell culture.

The technology of the present disclosure has been made in view of the above-described points, and an object thereof is to prevent the foreign matter from being mixed into the cell or the sample for cell culture during the processing of handling the cell or the sample for cell culture.

A processing device according to the technology of the present disclosure is a processing device for performing processing of handling a cell or a sample for cell culture by using a holding member including at least one holding section that holds the cell or the sample for cell culture. The processing device includes a protective surface that faces the holding section. The protective surface has an area larger than a planar area of a compartment in which the holding section is disposed, and includes a first fixing structure for fixing an object that accesses the holding section for the processing.

It is preferable that a space formed below the protective surface is capable of accommodating an entire holding member. The first fixing structure may include a groove or a notch that engages the object with the protective surface. The holding member may include a plurality of the holding sections arranged along a predetermined direction. In this case, a plurality of the grooves or a plurality of the notches may be provided at intervals corresponding to the arrangement of the plurality of holding sections. The first fixing structure may be provided on an edge of the protective surface along an arrangement direction of the holding sections. The processing device may further include an auxiliary section including a second fixing structure that holds the object between the first fixing structure and the second fixing structure.

The holding member may include a plurality of the holding sections arranged along a predetermined direction. In this case, one of the protective surface or the holding section may be movable relative to the other in an arrangement direction of the plurality of holding sections. The processing device may include a positioning mechanism for positioning a relative position between the protective surface and the holding section in a movement direction of the relative movement. The positioning mechanism may include a protrusion connected to the protective surface, and a guide provided along the movement direction and including a plurality of recesses that engage with the protrusion. The plurality of recesses may be provided at intervals corresponding to the arrangement of the plurality of holding sections.

The holding member may include a plurality of the holding sections arranged along a first direction and a second direction intersecting the first direction. In this case, the first fixing structure may include a plurality of grooves or a plurality of notches provided on an edge along the first direction at intervals corresponding to the arrangement of the plurality of holding sections in the first direction. One of the protective surface or the holding section may be movable relative to the other in the second direction.

The processing device may further include a base section on which the holding member is mounted. In this case, one of the protective surface or the base section may be movable relative to the other in the arrangement direction of the plurality of holding sections.

A processing method according to the technology of the present disclosure is a processing method for performing processing of handling a cell or a sample for cell culture by using a holding member including at least one holding section that holds the cell or the sample for cell culture. The processing method includes disposing a protective surface at a position facing the holding section, and performing the processing in a state in which a relative position between an object that accesses the holding section for the processing and the protective surface is fixed. The protective surface has an area larger than a planar area of a compartment in which the holding section is disposed.

According to the technology of the present disclosure, it is possible to prevent the foreign matter from being mixed into the cell or the sample for cell culture during the processing of handling the cell or the sample for cell culture.

### BRIEF DESCRIPTION OF THE DRAWINGS

FIG. 1 is a perspective view illustrating an example of a configuration of a processing device according to an embodiment of the technology of the present disclosure.
FIG. 2 is a perspective view illustrating an example of a configuration of a holding member according to the embodiment of the technology of the present disclosure.
FIG. 3A is a view illustrating an example of a method of forming a pattern of a cell or a cell adhesive on a culture surface of a culture substrate by using the holding member according to the embodiment of the technology of the present disclosure.
FIG. 3B is a view illustrating an example of the method of forming the pattern of the cell or the cell adhesive on the culture surface of the culture substrate by using the holding member according to the embodiment of the technology of the present disclosure.
FIG. 3C is a view illustrating an example of the method of forming the pattern of the cell or the cell adhesive on the culture surface of the culture substrate by using the holding member according to the embodiment of the technology of the present disclosure.
FIG. 3D is a view illustrating an example of the method of forming the pattern of the cell or the cell adhesive on the culture surface of the culture substrate by using the holding member according to the embodiment of the technology of the present disclosure.
FIG. 3E is a view illustrating an example of the method of forming the pattern of the cell or the cell adhesive on the culture surface of the culture substrate by using the holding member according to the embodiment of the technology of the present disclosure.
FIG. 3F is a view illustrating an example of the method of forming the pattern of the cell or the cell adhesive on the culture surface of the culture substrate by using the holding member according to the embodiment of the technology of the present disclosure.
FIG. 3G is a view illustrating an example of the method of forming the pattern of the cell or the cell adhesive on the culture surface of the culture substrate by using the holding member according to the embodiment of the technology of the present disclosure.
FIG. 4 is a perspective view illustrating a configuration of a base section alone according to the embodiment of the technology of the present disclosure.
FIG. 5 is a perspective view illustrating a configuration of a cover section alone according to the embodiment of the technology of the present disclosure.
FIG. 6 is a perspective view illustrating a configuration of an auxiliary section alone according to the embodiment of the technology of the present disclosure.
FIG. 7 is an X-Z plane view of the cover section and the base section as viewed from an open end side of the cover section according to the embodiment of the technology of the present disclosure.
FIG. 8 is a Y-Z plane view illustrating a wheel that moves on a guide according to the embodiment of the technology of the present disclosure.
FIG. 9A is an X-Y plane view illustrating an example of a processing method using the processing device according to the embodiment of the technology of the present disclosure.
FIG. 9B is an X-Y plane view illustrating an example of the processing method using the processing device according to the embodiment of the technology of the present disclosure.
FIG. 9C is an X-Y plane view illustrating an example of the processing method using the processing device according to the embodiment of the technology of the present disclosure.
FIG. 9D is an X-Y plane view illustrating an example of the processing method using the processing device according to the embodiment of the technology of the present disclosure.

### DESCRIPTION OF THE PREFERRED EMBODIMENTS

Hereinafter, an example of an embodiment of the present invention will be described with reference to the accompanying drawings. The same or equivalent components and portions in the drawings are denoted by the same reference numerals, and the overlapping description will be omitted.

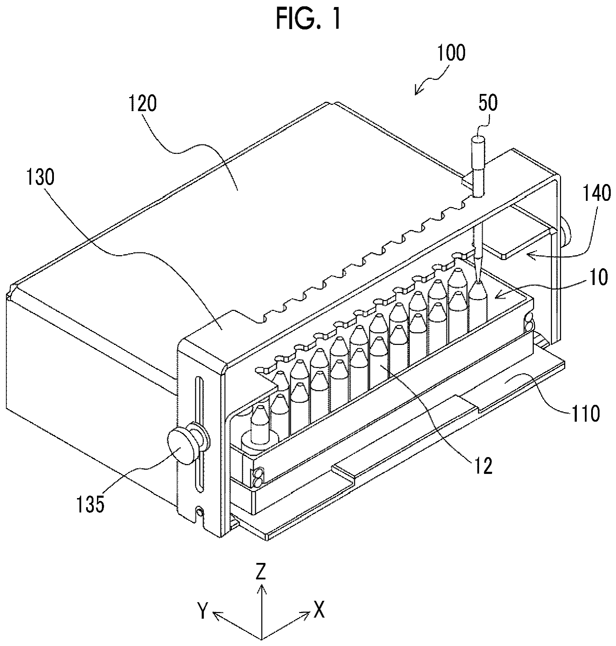

FIG. 1 is a perspective view illustrating an example of a configuration of a processing device 100 according to the embodiment of the technology of the present disclosure. The processing device 100 is used for performing processing of handling a cell or a sample for cell culture by using a holding member 10 including at least one holding section that holds the cell or the sample for cell culture. A type of the cell is not particularly limited, but may be, for example, human-derived induced pluripotent stem cells (iPS cells). The sample for cell culture is not particularly limited, but includes, for example, water, a culture medium, an artificial synthetic peptide solution, and a cell adhesive.

Prior to the description of the processing device 100 according to the present embodiment, the holding member 10 used together with the processing device 100 will be described.

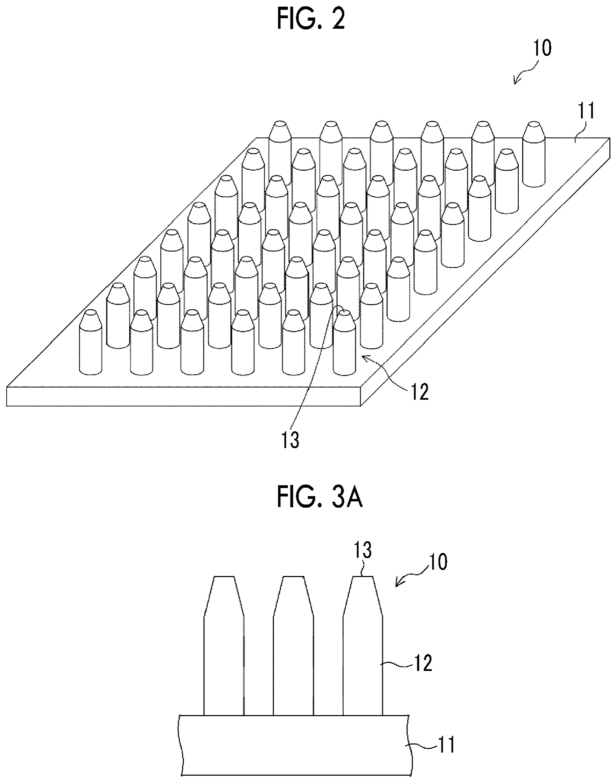

FIG. 2 is a perspective view illustrating an example of a configuration of the holding member 10. The holding member 10 is a device for forming a pattern of the cell or the cell adhesive on a culture surface of a culture substrate (not illustrated). The cells include cell aggregates such as colonies in which the cells are aggregated. The holding member 10 includes a plurality of columnar structures 12 arranged on a surface of a substrate 11. The columnar structure 12 is an example of a "holding section" according to the technology of the present disclosure. Each columnar structure 12 is a column whose axial direction is perpendicular to a main surface of the substrate 11. A length of the columnar structure 12 in the axial direction is not particularly limited, but may be, for example, equal to or more than 1 mm and equal to or less than 100 mm.

The culture substrate is a substrate on which the pattern of the cell or the cell adhesive is formed by the holding member 10, and is a substrate having the culture surface on which the cell is cultured. The culture substrate may, for example, be a well plate having a plurality of wells, and the culture surface may be, for example, a bottom surface of each of the plurality of wells provided in the well plate. The plurality of columnar structures 12 may be provided corresponding to the plurality of wells provided in the well plate. FIG. 1 illustrates a configuration comprising forty-eight columnar structures 12 provided corresponding to the respective wells in the well plate having forty-eight (6 × 8) wells. An arrangement form (the number of rows and columns and an arrangement interval) of the columnar structures 12 is not limited to the form illustrated in FIG. 1 and is determined as appropriate in accordance with the arrangement of the cell pattern to be formed on the culture substrate.

Each of the columnar structures 12 has a holding surface 13 for holding liquid at a distal end thereof. The liquid held on the holding surface 13 is typically a cell suspension or a cell adhesive. The pattern formed on the culture surface of the culture substrate by the holding member 10 reflects the planar shape of the holding surface 13. FIG. 1 illustrates the holding surface 13 having a circular planar shape. The shape of the holding surface 13 is determined as appropriate in accordance with the pattern of the cell or the cell adhesive to be formed on the culture substrate.

The columnar structure 12 is preferably configured such that at least the holding surface 13 is made of a hydrophobic (water-repellent) material. By forming the holding surface 13 from a hydrophobic (water-repellent) material, an amount of liquid that can be held on the holding surface 13 can be increased. Accordingly, a relatively large pattern of the cells or the cell adhesive can be formed on the culture surface of the culture substrate. It is preferable that the holding surface 13 of the columnar structure 12 be made of a material having a contact angle with water of equal to or greater than 45°. It is also preferable that the columnar structure 12 have resistance to autoclave sterilization, that is, heat resistance. The columnar structure 12 may be configured such that at least the holding surface 13 is made of a resin. For example, polyacetal can be suitably used as the material of the columnar structure 12 (holding surface 13). The columnar structure 12 may be made of the same material as the substrate 11 and integrated with the substrate 11.

Hereinafter, a method of forming the pattern of the cell or the cell adhesive on the culture surface of the culture substrate by using the holding member 10 will be described with reference to FIGS. 3A to 3G. First, the holding member 10 is installed such that the holding surface 13 of the columnar structure 12 faces vertically upward. In this case, the holding surface 13 is horizontal (FIG. 3A).

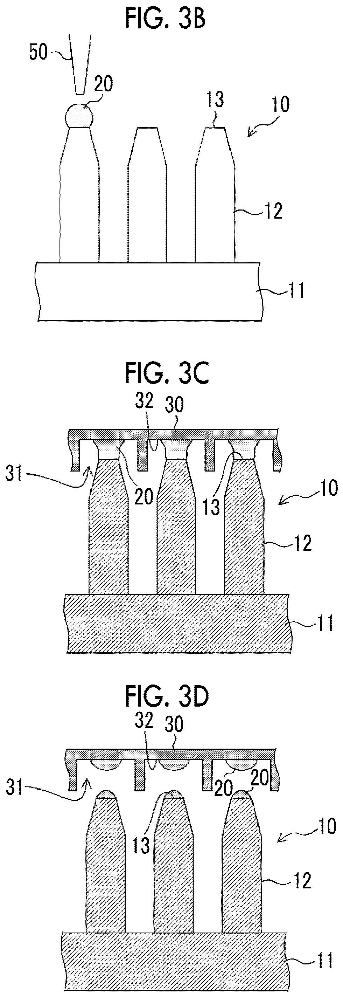

Next, with the holding surface 13 facing vertically upward, the cell suspension or the cell adhesive is dropped onto the respective holding surfaces 13 of the columnar structures 12 to form liquid droplets 20 of the cell suspension or the cell adhesive so as to establish a gas-liquid-solid interface along an outer edge of each holding surface 13 (FIG. 3B). Dropping the cell suspension or the cell adhesive onto the holding surface 13 is performed using a pipette 50 and the processing device 100 (see FIG. 1). The configuration and the method of use of the processing device 100 will be described later. The cell suspension or the cell adhesive is an example of "a cell or a sample for cell culture" according to the technology of the present disclosure. The pipette 50 is an example of an "object that accesses the holding section" according to the technology of the present disclosure.

A size of the pattern of the cell or the cell adhesive formed on the culture surface of the culture substrate can be controlled by an amount of liquid droplets 20 formed on the holding surface 13 of the columnar structure 12. The amount of liquid droplets 20 formed on the holding surface 13 is determined in accordance with the size of the pattern to be formed on a culture surface 32. By forming the liquid droplets 20 by dropping the liquid onto the holding surface 13, a "pinning effect" that keeps the liquid droplets 20 on the holding surface 13 can be produced. Owing to the "pinning effect," liquid droplets whose contours extend outward beyond the outer edge of the holding surface 13 can be held on the holding surface 13. The "pinning effect" also stabilizes a state of the liquid droplets 20 formed on the holding surface 13. Consequently, the state of the liquid droplets 20 formed on the holding surfaces 13 can be made uniform among the plurality of columnar structures 12, and, in turn, the pattern of the cell or the cell adhesive formed on the culture surface of the culture substrate can be made uniform.

Next, the liquid droplets 20 are brought into contact with the culture surface 32 of a culture substrate 30 in a state in which the holding surface 13 on which the liquid droplets 20 are formed faces vertically upward (FIG. 3C). That is, the culture substrate 30 is placed over the columnar structures 12 from above, thereby bringing the liquid droplets 20 into contact with the culture surface 32. As a result, the cell suspension or cell adhesive held on the holding surface 13 adheres to the culture surface 32 of the culture substrate 30. The culture substrate 30 may be a well plate having a plurality of wells 31. In this case, a bottom surface of each of the wells 31 is the culture surface 32. The plurality of columnar structures 12 provided in the holding member 10 respectively correspond to the plurality of wells 31 provided in the culture substrate 30.

In a case in which the liquid droplets 20 formed on the holding surface 13 are brought into contact with the culture surface 32, the columnar structures 12 and the wells 31 are aligned in a horizontal direction in a state in which the wells 31 are oriented with the bottom surfaces thereof facing downward. Thereafter, while maintaining the culture surface 32 horizontally, the culture surface 32 is gradually brought toward the holding surface 13 such that, in a batch operation, each of the liquid droplets 20 formed on the respective holding surfaces 13 of the plurality of columnar structures 12 is brought into contact with the bottom surfaces (culture surface 32) of the plurality of wells 31.

The size of the pattern formed on the culture surface 32 can be controlled by a distance between the holding surface 13 and the culture surface 32 in a case in which the liquid droplets 20 are brought into contact with the culture surface 32. The distance between the holding surface 13 and the culture surface 32 in a case in which the liquid droplets 20 are brought into contact with the culture surface 32 is set in accordance with the size of the pattern to be formed on the culture surface 32.

After bringing the liquid droplets 20 into contact with the culture surface 32, the culture surface 32 is moved away from the holding surface 13. As a result, the liquid droplets 20 are divided into portions on the holding surface 13 side and the culture surface 32 side (FIG. 3D). On the bottom surface (culture surface 32) of each of the plurality of wells 31, the pattern of the cell suspension or cell adhesive reflecting the planar shape of the holding surface 13 is formed in a batch operation. Hereinafter, as an example, a case will be described in which the pattern of the cell adhesives is formed on the bottom surface (culture surface 32) of each well 31. Extracellular matrix proteins such as vitronectin, laminin, fibronectin, collagens, and gelatins may be used as the cell adhesive.

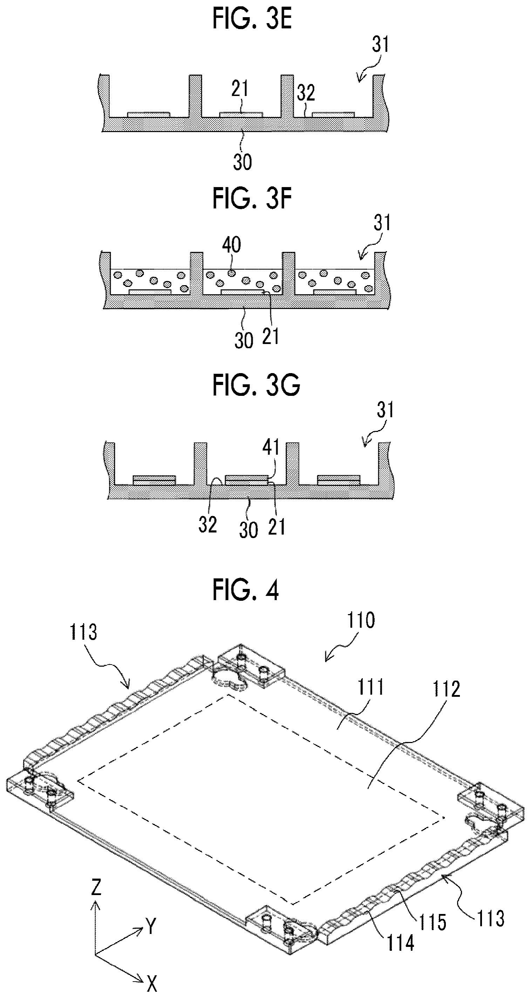

After the cell adhesive 21 is adhered to the culture surface 32, the excess of the cell adhesive 21 is removed from each well 31. The cell adhesive 21 adhered to the bottom surface (culture surface 32) of each well 31 remains on the culture surface 32 while maintaining the pattern (FIG. 3E).

Next, cells 40 are seeded onto the culture surface 32 (FIG. 3F). Seeding of the cells 40 is performed by dispensing a cell suspension adjusted to a predetermined cell concentration into each well 31. The cells 40 need only be adherent cells, and the type of the cells 40 is not particularly limited. The cells 40 may be, for example, human-derived iPS cells.

The cells 40 adhere to a region coated with the cell adhesive 21 on the culture surface 32. Thereafter, by culturing the cells 40 under appropriate conditions, the cells 40 proliferate in the regions coated with the cell adhesive 21. As a result, a cell pattern 41 corresponding to the pattern of the cell adhesive 21 is formed on the culture surface 32 (FIG. 3G).

With the holding member 10 and the pattern forming method, the pattern having the size corresponding to the size of the liquid droplets 20 formed on the holding surface 13 is formed, so that a pattern having a relatively large size can be formed.

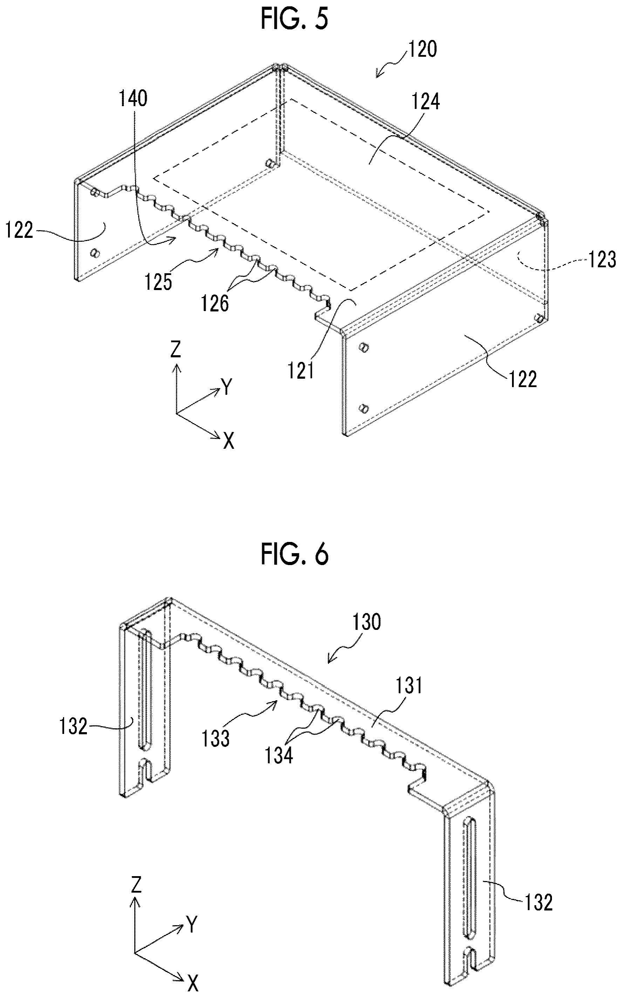

Hereinafter, the processing device 100 will be described. The processing device 100 is used, for example, in a process (see FIG. 3B) of forming the liquid droplets 20 on the holding surface 13 of the holding member 10 by using the pipette 50. As illustrated in FIG. 1, the processing device 100 includes a base section 110, a cover section 120, and an auxiliary section 130. FIG. 4 is a perspective view illustrating a configuration of the base section 110 alone. FIG. 5 is a perspective view illustrating a configuration of the cover section 120 alone. FIG. 6 is a perspective view illustrating a configuration of the auxiliary section 130 alone.

The base section 110 is a flat plate-like member and has a mounting surface 111 on which the holding member 10 is mounted. A region 112 surrounded by a dotted line in FIG. 4 is a region in which the holding member 10 is mounted. An area of the mounting surface 111 is larger than a planar area of the holding member 10, and an entire holding member 10 can be mounted on the mounting surface 111.

The cover section 120 has a rectangular box-like structure formed by a protective surface 121, a side surface 122, and a rear surface 123. The protective surface 121 is a portion corresponding to an upper surface of the rectangular box, and a portion corresponding to a front surface and a bottom surface of the rectangular box is open. In a state in which the holding member 10 is mounted on the mounting surface 111 of the base section 110, the protective surface 121 is located above the holding member 10, the side surface 122 is located to the side of the holding member 10, and the rear surface 123 is located behind the holding member 10.

The protective surface 121 is parallel to the mounting surface 111 of the base section 110. That is, in a state in which the holding member 10 is mounted on the base section 110, the protective surface 121 faces the holding surface 13 of the columnar structure 12. The side surface 122 and the rear surface 123 are perpendicular to the mounting surface 111 of the base section 110.

The protective surface 121 has an area larger than a planar area of a compartment in which the columnar structure 12 of the holding member 10 is disposed. An area of a region 124 surrounded by a dotted line in FIG. 5 corresponds to the planar area of the compartment in which the columnar structure 12 of the holding member 10 is disposed. The entire holding member 10 can be accommodated in a space 140 (that is, a space formed below the protective surface 121) surrounded by the protective surface 121, the side surface 122, the rear surface 123 of the cover section 120, and the mounting surface 111 of the base section 110. By disposing the protective surface 121 above the columnar structure 12, the liquid droplets 20 (the cell or the sample for cell culture) held on the holding surface 13 of the columnar structure 12 can be protected from foreign matter that falls from above. As a result, it is possible to prevent the foreign matter from being mixed into the liquid droplets 20 (the cell or the sample for cell culture).

A shape of the protective surface 121 is a rectangle or a square having an edge along an X direction and an edge along a Y direction. The holding member 10 is mounted on the base section 110 such that an arrangement direction of the columnar structures 12 is along the X direction and the Y direction (that is, parallel to each edge of the protective surface 121).

The protective surface 121 includes a first fixing structure 125 for fixing the pipette 50 to an edge of the cover section 120 along the X direction (arrangement direction of the columnar structures 12) on the open end side (front side). The first fixing structure 125 includes a plurality of grooves (or notches) 126 provided along the X direction. A plurality of pipettes 50 are fixed in a state of being positioned with respect to the holding surface 13 by engaging with the plurality of grooves (or notches) 126.

FIG. 1 illustrates a state in which only one pipette 50 is fixed to the protective surface 121, but the plurality of pipettes 50 can be simultaneously fixed to the protective surface 121 by engaging the plurality of pipettes 50 with each of the plurality of grooves (or notches) 126. The plurality of grooves (or notches) 126 are provided at the same interval as an arrangement interval of the columnar structures 12 in the X direction. By engaging the plurality of pipettes 50 with the plurality of grooves (or notches) 126, each of the pipettes 50 is disposed at a position corresponding to each of the columnar structures 12 arranged along the X direction.

The auxiliary section 130 is a member for assisting in fixing the pipette 50 to the protective surface 121. The auxiliary section 130 includes a first portion 131 that extends along the X direction, and a second portion 132 that is connected to both ends of the first portion 131 in the X direction and extends along the Z direction.

The second portion 132 is fastened to the side surface of the cover section 120 by a screw 135 (see FIG. 1), so that the first portion 131 is supported to be located above the first fixing structure 125 of the protective surface 121. The first portion 131 includes a second fixing structure 133 that holds the pipette 50 between the first fixing structure 125 provided on the protective surface 121 and the second fixing structure 133.

The second fixing structure 133 includes a plurality of grooves (or notches) 134 provided along the X direction, as in the first fixing structure 125. The plurality of grooves (or notches) 134 constituting the second fixing structure 133 are provided at the same interval as the arrangement interval of the columnar structures 12 in the X direction, as in the plurality of grooves (or notches) 126 constituting the first fixing structure 125, and are provided at positions corresponding to the plurality of grooves (or notches) 126 constituting the first fixing structure 125.

In a case in which the pipette 50 engages with the groove (or notch) 126 constituting the first fixing structure 125 and engages with the groove (or notch) 134 constituting the second fixing structure 133, as illustrated in FIG. 1, the pipette 50 is in a state of being held between the first fixing structure 125 and the second fixing structure 133.

One of the cover section 120 or the base section 110 is movable relative to the other in the Y direction. That is, in a state in which the holding member 10 is mounted on the base section 110, one of the protective surface 121 or the columnar structure 12 is movable relative to the other in the arrangement direction of the columnar structure 12. In the present embodiment, the base section 110 and the holding member 10 are maintained in a stationary state, and the cover section 120 and the auxiliary section 130 move in the Y direction. The pipette 50 moves together with the cover section 120 and the auxiliary section 130. The cover section 120, the auxiliary section 130, and the pipette 50 may be maintained in a stationary state, and the base section 110 and the holding member 10 may move in the Y direction.

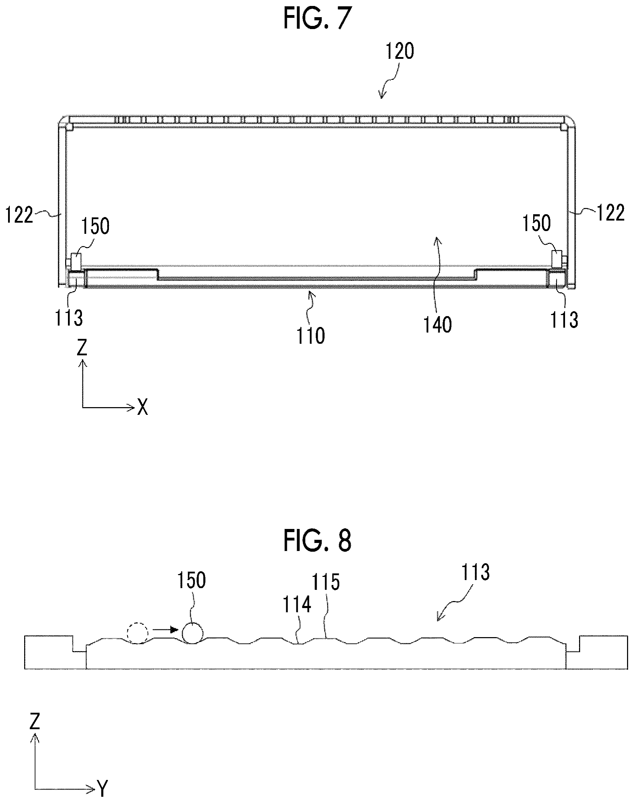

The processing device 100 includes a positioning mechanism for positioning a relative position between the cover section 120 and the base section 110 (in other words, a relative position between the protective surface 121 and the columnar structure 12) in a movement direction of the relative movement. The positioning mechanism includes a wheel 150 (see FIG. 7) connected to the protective surface 121 and a plurality of recesses 114 (see FIGS. 4 and 8) that engage with the wheel 150.

FIG. 7 is an X-Z plane view of the cover section 120 and the base section 110 as viewed from the open end side of the cover section 120. The cover section 120 includes the wheels 150 attached to each of two side surfaces 122. The wheels 150 are disposed inside the space 140 surrounded by the protective surface 121 of the cover section 120, the side surface 122, and the mounting surface 111 of the base section 110.

Meanwhile, as illustrated in FIG. 4, the base section 110 includes guides 113 that extend along the Y direction, which is the movement direction of the cover section 120, at both ends of the base section 110 in the X direction. The guide 113 includes the plurality of recesses 114 and a plurality of protrusions 115 provided along the Y direction. That is, the guide 113 has a recess-and-protrusion structure in which the recesses 114 and the protrusions 115 are continuous along the Y direction. As illustrated in FIG. 8, the cover section 120 moves in the Y direction by rolling the wheel 150 on the guide 113. In a case in which the wheels 150 are engaged with the recesses 114, the wheels 150 are held between the protrusions 115 at the front and rear, and the movement of the wheels 150 in the Y direction is suppressed. As a result, the movement of the cover section 120 in the Y direction can be performed stepwise, and the relative position between the cover section 120 and the base section 110 in the Y direction can be positioned.

The plurality of recesses 114 are provided at the same interval as the arrangement interval of the columnar structures 12 in the Y direction. As a result, the position of the pipette 50 that moves together with the cover section 120 and the auxiliary section 130 in the Y direction can be made to correspond to the arrangement of the columnar structures 12 in the Y direction. That is, in a state in which the wheel 150 engages with a certain recess 114, the pipette 50 is positioned in an n-th column of the columnar structure 12 in the Y direction, and in a state in which the wheel 150 engages with the adjacent recess 114, the pipette 50 is positioned in an (n+1)-th column of the columnar structure 12 in the Y direction.

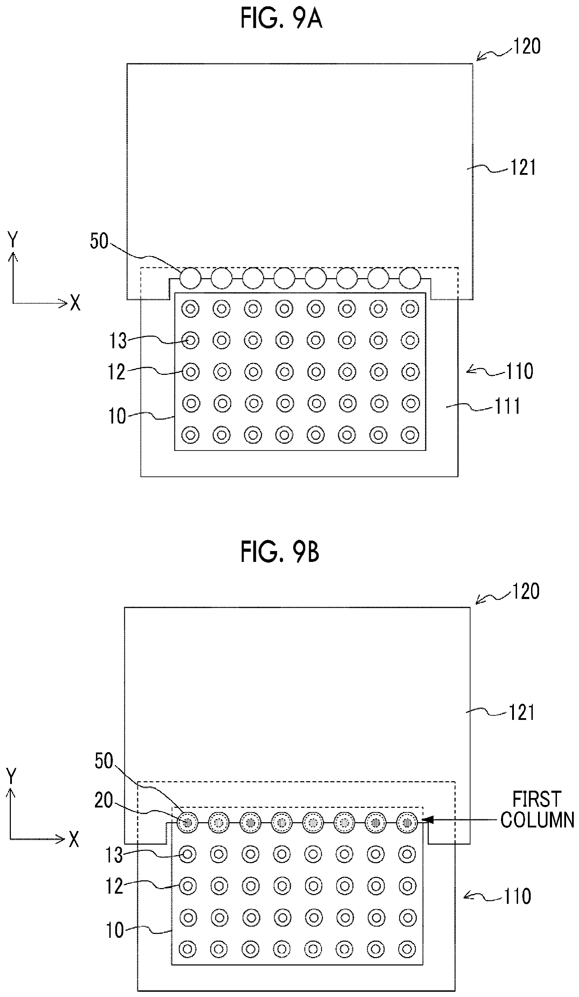

Hereinafter, a processing method using the processing device 100 will be described. FIGS. 9A to 9D are X-Y plane views illustrating examples of the processing method using the processing device 100. In FIGS. 9A to 9D, the auxiliary section 130 is not illustrated.

First, the pipette 50 engages with each of the grooves (or notches) 126 constituting the first fixing structure 125 provided on the protective surface 121. Then, each of the pipettes 50 engages with each of the grooves (or notches) 134 constituting the second fixing structure 133 provided in the auxiliary section 130 to assist in fixing each of the pipettes 50 to the protective surface 121 (FIG. 9A).

Next, the holding member 10 including the plurality of columnar structures 12 is mounted on the mounting surface 111 of the base section 110 (FIG. 9A).

Next, the cover section 120 is moved one step in the Y direction. Each of the pipettes 50 moves integrally with the cover section 120. As a result, each of the pipettes 50 is positioned at the position corresponding to each of the columnar structures 12 arranged in the first column in the Y direction. That is, each of the columnar structures 12 arranged in a first column in the Y direction is disposed directly below an outlet of the corresponding pipette 50. Thereafter, a liquid containing the cell or the sample for cell culture is discharged from each of the pipettes 50. As a result, the liquid droplets 20 containing the cell or the sample for cell culture are formed on each of the holding surfaces 13 of the columnar structures 12 arranged in the first column in the Y direction by a batch operation (FIG. 9B).

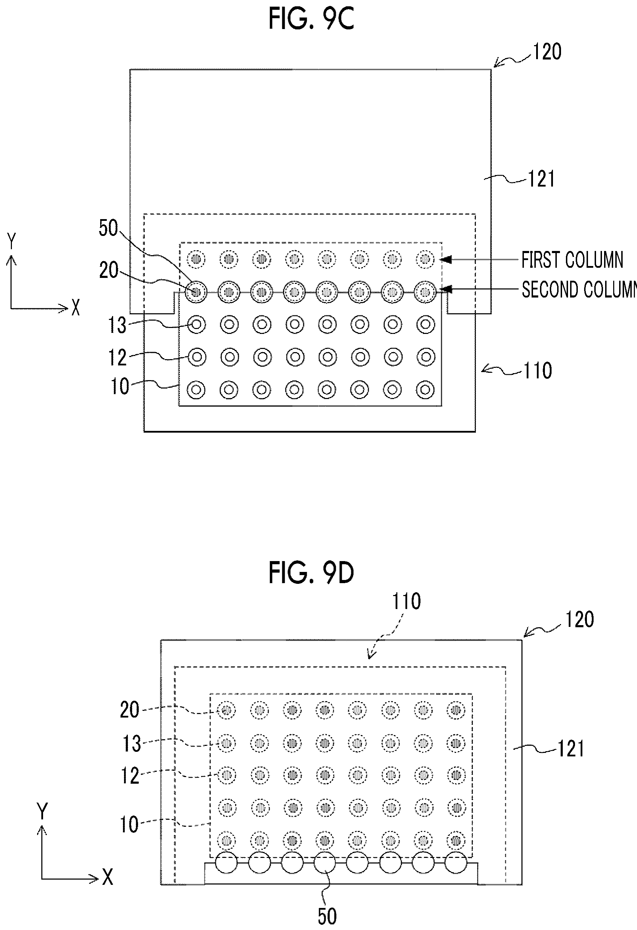

Next, the cover section 120 is further moved one step in the Y direction. Each of the pipettes 50 moves integrally with the cover section 120. As a result, each of the pipettes 50 is positioned at the position corresponding to each of the columnar structures 12 arranged in a second column in the Y direction. Each of the columnar structures 12 arranged in the first column in the Y direction is covered by the protective surface 121. As a result, the foreign matter is prevented from being mixed into the liquid droplets 20 held on each of the holding surfaces 13 of the columnar structures 12 arranged in the first column in the Y direction. Thereafter, a liquid containing the cell or the sample for cell culture is discharged from each of the pipettes 50. As a result, the liquid droplets 20 containing the cell or the sample for cell culture are formed on each of the holding surfaces 13 of the columnar structures 12 arranged in the second column in the Y direction by a batch operation (FIG. 9C).

Thereafter, the cover section 120 is moved step by step in the Y direction, and the liquid droplets 20 are sequentially formed on each of the holding surfaces 13 of the columnar structures 12 arranged in each column. Each of the columnar structures 12 is covered by the protective surface 121 by the movement of the cover section 120 after the liquid droplets 20 are formed. As a result, the foreign matter is prevented from being mixed into the liquid droplets 20 (FIG. 9D).

As described above, with the processing device 100 according to the embodiment of the technology of the present disclosure and the processing method using the processing device 100, each of the columnar structures 12 is covered by the protective surface 121 after the liquid droplets 20 containing the cell or the sample for cell culture are formed. Therefore, with the processing device 100 according to the embodiment of the technology of the present disclosure and the processing method using the processing device 100, it is possible to prevent the foreign matter from being mixed into the cell or the sample for cell culture during the processing of handling the cell or the sample for cell culture by using the holding member 10 including the plurality of columnar structures 12 that hold the cell or the sample for cell culture.

In the above description, as an example, a case has been described in which the device comprising the plurality of columnar structures 12 as the holding section that holds the cell or the sample for cell culture is used as the holding member, but the technology of the present disclosure is not limited to this aspect. A plate or a dish comprising the plurality of wells as the holding sections that hold the cell or the sample for cell culture can also be used as the holding member. In this case, the processing device 100 is used, for example, for processing of dropping the liquid droplets of the cell or the sample for cell culture into each well. That is, the processing device 100 can also be used for the processing illustrated in FIG. 3F. Each well is covered by the protective surface 121 that moves integrally with the pipette after the liquid droplets containing the cell or the sample for cell culture are dropped. In addition, the technology of the present disclosure can also be applied to the holding member 10 comprising at least one holding section such as the columnar structure and the well that holds the cell or the sample for cell culture.

In addition, in the above description, as an example, a case has been described in which the pipette 50 is used as the object that accesses the holding section for the processing of handling the cell or the sample for cell culture, but the technology of the present disclosure is not limited to this aspect. The technology of the present disclosure can also be applied to a case in which the object that accesses the holding section for processing to handle the cell or the sample for cell culture is, for example, a lifter, a spreader, or various sensors.

In addition, in the above description, as an example, a configuration has been described in which the protective surface 121 is supported by the side surface 122 and the rear surface 123 of the cover section 120, but the technology of the present disclosure is not limited to this aspect. For example, a configuration may be adopted in which the protective surface 121 is supported by a support section that extends from above the protective surface 121 in the vertical direction toward the protective surface 121. In addition, a configuration may be adopted in which the protective surface 121 is supported by a support section that extends from the side of the protective surface 121 in the horizontal direction toward the protective surface 121.

In addition, in the above description, as an example, a configuration has been described in which the first fixing structure 125 is provided on the edge of the protective surface 121 along the arrangement direction of the columnar structure 12, but the technology of the present disclosure is not limited to this aspect. The first fixing structure 125 may be a structure that is different from the protective surface 121 but connected to the protective surface 121.

In addition, in the above description, as an example, a configuration has been described in which the positioning mechanism includes the wheel 150 (see FIG. 7) connected to the protective surface 121 and the plurality of recesses 114 (see FIGS. 4 and 8) that engage with the wheel 150, but the technology of the present disclosure is not limited to this aspect. The portion that engages with the recess 114 need not be a portion that performs a rotational movement such as the wheel 150. That is, the positioning mechanism may be composed of a protrusion connected to the protective surface 121 and a plurality of recesses 114 that engage with the protrusion.

In regard to the embodiment described above, the following supplementary notes are further disclosed.

### (Supplementary Note 1)

A processing device for performing processing of handling a cell or a sample for cell culture by using a holding member including at least one holding section that holds the cell or the sample for cell culture, the processing device including: a protective surface that faces the holding section, in which the protective surface has an area larger than a planar area of a compartment in which the holding section is disposed, and includes a first fixing structure for fixing an object that accesses the holding section for the processing.

### (Supplementary Note 2)

The processing device according to supplementary note 1, in which a space formed below the protective surface is capable of accommodating an entire holding member.

### (Supplementary Note 3)

The processing device according to supplementary note 1 or 2, in which the first fixing structure includes a groove or a notch that engages the object with the protective surface.

### (Supplementary Note 4)

The processing device according to supplementary note 3, in which the holding member includes a plurality of the holding sections arranged along a predetermined direction, and a plurality of the grooves or a plurality of the notches are provided at intervals corresponding to the arrangement of the plurality of holding sections.

### (Supplementary Note 5)

The processing device according to any one of supplementary notes 1 to 4, in which the first fixing structure is provided on an edge of the protective surface along an arrangement direction of the holding sections.

### (Supplementary Note 6)

The processing device according to any one of supplementary notes 1 to 5, further including: an auxiliary section including a second fixing structure that holds the object between the first fixing structure and the second fixing structure.

### (Supplementary Note 7)

The processing device according to any one of supplementary notes 1 to 6, in which the holding member includes a plurality of the holding sections arranged along a predetermined direction, and one of the protective surface or the holding section is movable relative to the other in an arrangement direction of the plurality of holding sections.

### (Supplementary Note 8)

The processing device according to supplementary note 7, further including: a positioning mechanism for positioning a relative position between the protective surface and the holding section in a movement direction of the relative movement.

### (Supplementary Note 9)

The processing device according to supplementary note 8, in which the positioning mechanism includes a wheel connected to the protective surface, and a guide provided along the movement direction and including a plurality of recesses that engage with the wheel.

### (Supplementary Note 10)

The processing device according to supplementary note 9, in which the plurality of recesses are provided at intervals corresponding to the arrangement of the plurality of holding sections.

### (Supplementary Note 11)

The processing device according to any one of supplementary notes 1 to 10, in which the holding member includes a plurality of the holding sections arranged along a first direction and a second direction intersecting the first direction, the first fixing structure includes a plurality of grooves or a plurality of notches provided on an edge along the first direction at intervals corresponding to the arrangement of the plurality of holding sections in the first direction, and one of the protective surface or the holding section is movable relative to the other in the second direction.

### (Supplementary Note 12)

The processing device according to supplementary note 7, further including: a base section on which the holding member is mounted, in which one of the protective surface or the base section is movable relative to the other in the arrangement direction of the plurality of holding sections.

### (Supplementary Note 13)

A processing method for performing processing of handling a cell or a sample for cell culture by using a holding member including at least one holding section that holds the cell or the sample for cell culture, the processing method including: disposing a protective surface at a position facing the holding section; and performing the processing in a state in which a relative position between an object that accesses the holding section for the processing and the protective surface is fixed, in which the protective surface has an area larger than a planar area of a compartment in which the holding section is disposed.

It should be noted that the disclosure of Japanese Patent Application No. 2023-111076, filed on July 5, 2023 is incorporated in the present specification in its entirety by reference. All of the documents, the patent applications, and the technical standards described in the present specification are incorporated herein by reference to the same extent as in a case in which each of the documents, the patent applications, and the technical standards are specifically and individually described by being incorporated in the present specification by reference.

## Claims

1. A processing device for performing processing of handling a cell or a sample for cell culture by using a holding member including at least one holding section that holds the cell or the sample for cell culture, the processing device comprising:
a protective surface that faces the holding section,
wherein the protective surface has an area larger than a planar area of a compartment in which the holding section is disposed, and includes a first fixing structure for fixing an object that accesses the holding section for the processing.

2. The processing device according to claim 1,
wherein a space formed below the protective surface is capable of accommodating an entire holding member.

3. The processing device according to claim 1,
wherein the first fixing structure includes a groove or a notch that engages the object with the protective surface.

4. The processing device according to claim 3,
wherein the holding member includes a plurality of the holding sections arranged along a predetermined direction, and
a plurality of the grooves or a plurality of the notches are provided at intervals corresponding to the arrangement of the plurality of holding sections.

5. The processing device according to claim 1,
wherein the first fixing structure is provided on an edge of the protective surface along an arrangement direction of the holding sections.

6. The processing device according to claim 1, further comprising:
an auxiliary section including a second fixing structure that holds the object between the first fixing structure and the second fixing structure.

7. The processing device according to claim 1,
wherein the holding member includes a plurality of the holding sections arranged along a predetermined direction, and
one of the protective surface or the holding section is movable relative to the other in an arrangement direction of the plurality of holding sections.

8. The processing device according to claim 7, further comprising:
a positioning mechanism for positioning a relative position between the protective surface and the holding section in a movement direction of the relative movement.

9. The processing device according to claim 8,
wherein the positioning mechanism includes
a protrusion connected to the protective surface, and
a guide provided along the movement direction and including a plurality of recesses that engage with the protrusion.

10. The processing device according to claim 9,
wherein the plurality of recesses are provided at intervals corresponding to the arrangement of the plurality of holding sections.

11. The processing device according to claim 1,
wherein the holding member includes a plurality of the holding sections arranged along a first direction and a second direction intersecting the first direction,
the first fixing structure includes a plurality of grooves or a plurality of notches provided on an edge along the first direction at intervals corresponding to the arrangement of the plurality of holding sections in the first direction, and
one of the protective surface or the holding section is movable relative to the other in the second direction.

12. The processing device according to claim 7, further comprising:
a base section on which the holding member is mounted,
wherein one of the protective surface or the base section is movable relative to the other in the arrangement direction of the plurality of holding sections.

13. A processing method for performing processing of handling a cell or a sample for cell culture by using a holding member including at least one holding section that holds the cell or the sample for cell culture, the processing method comprising:
disposing a protective surface at a position facing the holding section; and
performing the processing in a state in which a relative position between an object that accesses the holding section for the processing and the protective surface is fixed,
wherein the protective surface has an area larger than a planar area of a compartment in which the holding section is disposed.
